Europäisches Patentamt

European Patent Office

Office européen des brevets

(19)

(11) Veröffentlichungsnummer: **0 079 308**
**B1**

(12) **EUROPÄISCHE PATENTSCHRIFT**

(45) Veröffentlichungstag der Patentschrift:
25.09.85

(21) Anmeldenummer: 82810462.0

(22) Anmeldetag: 01.11.82

(51) Int. Cl.⁴: **A 01 N 37/34**, C 10 L 1/22,
C 10 M 133/24, D 21 C 9/00,
C 11 D 3/48, C 09 D 5/14,
C 02 F 1/50

(54) Verwendung von Mono- und Dibromdicyanomethan zur Bekämpfung von Schadorganismen und Mittel für die Bekämpfung von Schadorganismen.

(30) Priorität: 06.11.81 CH 7111/81

(43) Veröffentlichungstag der Anmeldung:
18.05.83 Patentblatt 83/20

(45) Bekanntmachung des Hinweises auf die Patenterteilung:
25.09.85 Patentblatt 85/39

(84) Benannte Vertragsstaaten:
BE DE FR GB IT NL

(56) Entgegenhaltungen:
US - A - 3 608 084
US - A - 3 877 922

Die Akte enthält technische Angaben, die nach dem Eingang der Anmeldung eingereicht wurden und die nicht in dieser Patentschrift enthalten sind.

(73) Patentinhaber: CIBA-GEIGY AG, Postfach,
CH-4002 Basel (CH)

(72) Erfinder: Grade, Reinhardt, Dr., Tulpenweg 11,
D-6140 Bensheim (DE)
Erfinder: Lorenz, Joachim, Dr., Im Tiefen Weg 17,
D-6140 Bensheim 3 (DE)

## Beschreibung

Die vorliegende Erfindung betrifft die Bekämpfung von Schadorganismen in Wasser und in Wasser enthaltenden Systemen oder in Ölen oder Treibstoffen unter Einsatz von Mono- oder Dibromdicyanomethan als Altivsubstanz.

Aus der britischen Pat.-Schrift 1 103 391 sind (Halogeno-Cyano) Acetylderivate als breit einsetzbare Pestizide bekannt. In der US-PS 2 359 266 wird Dichlormalonitril als Insektizid beschrieben. Die analoge Dibrom-Verbindung ist aus Beilstein E II 2.539 als Öl, das unlöslich in Wasser, aber wasserdampfflüchtig ist, bekannt.

Aus der US-PS 3 608 084 ist bekannt, dass gewisse halogenierte aliphatisch Nitrile, darunter auch Dibromcyanomethan, den Wuchs von Aerobacter Bakterien verbindern.

Die Erfindung betrifft die Verwendung von Mono- oder Dibromdicyanomethan zur Bekämpfung von Schadorganismen in Wasser, in Wasser enthaltenden Systemen, in Ölen oder in Treibstoffen.

Mono- und Dibromdicyanomethan sind in ausserordentlich geringen Konzentrationen wirksam und können vorzugsweise in Konzentrationen von 1-100 ppm und insbesondere 2-30 ppm eingesetzt werden.

Von besonderem Interesse ist die sehr gute Löslichkeit der Verbindungen in organischen inerten Lösungsmitteln. Dies erlaubt die Bereitstellung konzentrierter Lösungen, was besonders für deren Transport von grosser Bedeutung ist. Es ist überraschend, dass die als wasserdampf-flüchtig bekannte Dibromverbindung unter Bedingungen (z.B. in Kühltürmen), unter denen sie ausgetragen wird, ihre microbiocide Wirkung voll entfaltet. Dies macht deutlich, wie rasch die Verbindungen auch in kleinsten Konzentrationen wirksam sind.

Mono- und Dibromdicyanomethan zur Bekämpfung von Schadorganismen sind hoch wirksam gegen schädliche Mikroorganismen und zur Verhinderung von Schleimbildung. Die erfindungsgemässe Verwendung erfolgt in Wasser oder Wasser enthaltenden Systemen, insbesondere in Kühlkreisläufen und in anderen natürlichen und künstlichen, technischen wässrigen Materialien. Beispiele für solche Massen sind Schneid- und Bohröle, Papier- und Zellstoffmassen, wässrige Dispersionsfarben, Pigmentdispersionen, Agrarchemikalien, Leime und Stärkekleister. Mono- und Dibromdicyanomethan können auch in Ölen und Treibstoffen, z.B. Flugbenzin sowie bei der sekundären Ölgewinnung zur Bekämpfung von Schadorganismen eingesetzt werden.

Ein weiterer Gegenstand der vorliegenden Erfindung ist die Verwendung von Gemischen von mono- und Dibromdicyanomethan in beliebigem Verhältnis.

Die erfindungsgemässe Verwendung erfolgt in der Regel durch Applikation von flüssigen, pastenartigen oder festen Zubereitungen. Derartige Zubereitungen können z.B. Suspensionen, Emulsionen und Lösungen in organischen Lösungsmitteln sein.

Mono- und Dibromdicyanomethan können allein oder in Zubereitungen oder in Kombination mit anderen Biociden eingesetzt werden. Dazu sei insbesondere auf die folgenden Publikationen hingewiesen:

A. Simek et al «Antimicrobially active substances», Folia Microbiologica 14, 508-510 (1969); H.J. Hueck et al «Bacteriostatic fungistatic and algistatic activity of fatty nitrogen compounds», Appl. Microbiology 14, 308-319 (1966); A.T. Fuller «Antibacterial action and chemical constitution in long chain aliphatic bases», Biochem. J. 36, 548-558 (1942) und ferner H.D. Held, «Kühlwasser» S. 312 ff Vulkan-Verlag Essen (1977) und ferner auf DE 2 247 369 und US 4 070 400.

Werden die Verbindungen in Kombination mit andern Biociden eingesetzt, so sind insbesondere quaternäre Ammonium- oder Phosphoniumverbindungen, Amine, Isothiazolonderivate, Chlorphenole, Organozinnverbindungen, s-Triazine, Formaldehydabspaltende Produkte oder schwefelhaltige Biocide hervorzuheben.

Von besonderem Interesse können solche Kombinationen sein, die mit Verbindungen erfolgen, insbesondere dann, wenn diese Verbindungen vor allem wuchshemmende und weniger abtötende Wirkung besitzen.

Für die Herstellung der Mittel bzw. Zubereitungen gegen Schadstoffe können z.B. die folgenden Hilfsstoffe mitverwendet werden:
Trägerstoffe, Streckmittel, Emulgatoren, Anfeuchtmittel, Fixiermittel und grenzflächenaktive Mittel. Bei der Bekämpfung von Mikroorganismen in Weisswasser oder Schleimen, welche vor allem in der Papierindustrie betrieben werden muss, können die Formulierungen Detergentien enthalten und damit bestehende Schleime ablösen bzw. den Zugang des Bactericids zur Abtötung bestehender Schleime fördern. Beispiele von Trägersubstanzen sind Ton, Kaolin, Talkum, Diatomeenerde, Kieselsäure, Calciumcarbonat, Benzol, Alkohole, Ester, Xylol, Polyole, Polyäthylenoxid-addukte, Methylnaphthalin, Dimethylformamid, Diäthylsulfoxid, tierische und pflanzliche Öle, sowie Fettsäure und deren Ester. Bevorzugt werden Lösungsmittel als Trägerstoffe verwendet.

Ein weiterer Gegenstand der Erfindung sind auch Mittel zum Bekämpfen von Mikroorganismen, enthaltend Mono- oder Dibromdicyanomethan und einen für Mittel zur Bekämpfung von Schadorganismen üblichen geeigneten Hilfsstoff, wobei die Konzentration des Dibromdicyanomethan bzw. Monobromdicyanomethan, bezogen auf das gesamte Mittel, 0,1-80, vorzugsweise 0,5-20 Gew.-% beträgt.

*Beispiel*

*Abtötung von Pseudomonas aeruginosa*

Die im Caso-Pepton-Bouillon gewachsene ÜNK des Bakterienstammes

Pseudomonas aeruginosa ATCC 10145

wird in Saline 1/1000 verdünnt. Von dieser Suspension werden weiterhin in Tyrode eine 1/100 Verdünnung durchgeführt (Endverdünnung $10^{-5}$) und die Kultur 24 h bei 30°C im Schüttelwasserbad ( + Biocid 100 mg/l) bebrütet. Danach werden aus den Proben 5 $\mu$l entnommen und auf Caso-Pepton-Agar getropft. Nach einer erneuten Bebrütung bei 30°C für 24 h wird visuell nach Wuchs geprüft.

### Tabelle I

| Konzentration | Wuchs | |
| mg/l | $Br_2C(CN)_2$ | $BrCH(CN)_2$ |
|---|---|---|
| 2 | (—) | — |
| 5 | — | — |
| 10 | — | — |
| 30 | — | — |
| 60 | — | — |
| 100 | — | — |
| 0 | + | + |

+ = Wuchs, keine Abtötung
(—) = 1 - 10 Kolonien, schwache Abtötung
— = kein Wuchs, alle Zellen abgetötet

*Beispiel 2*

*Bestimmung der minimalen Abtötungskonzentration einer Bakterien-Mischkultur*

Von den in Caso-Pepton-Bouillon gewachsenen ÜNK's der verschiedenen Bakterienstämme

1 Escherichia coli
4 Bacillus cereus var. myciodes
6 Staphylococcus aureus
7 Pseudomonas aeruginosa
10 Enterobacter aerogenes
13 Proteus vulgaris

werden zur Herstellung der Mischkultur jeweils soviel in Tyrode gegeben, dass eine Endverdünnung von 1/1000 erreicht wurde, und die Mischkultur 24 h bei 30°C im Schüttelwasserbad bebrütet.

Danach werden aus den Proben 5 $\mu$l entnommen und auf Caso-Pepton-Agar getropft. Nach einer erneuten Bebrütung bei 30°C für 24 h wird visuell nach Wuchs ausgewertet.

### Tabelle II

| Konzentration | Wuchs | |
| (mg/l) | Monobrom-dicyanomethan | Dibrom-dicyanomethan |
|---|---|---|
| 2 | + | + |
| 5 | + | + |
| 10 | — | — |
| 30 | — | — |
| 60 | — | — |
| 100 | — | — |
| Kontrolle | + | + |

+ = Wuchs, keine Abtötung
— = kein Wuchs, alle Bakterien getötet

*Beispiel 3*

*Bestimmung der minimalen Abntötungskonzentration einer Bakterien mischkultur, verschiedene Keimzahl*

Der Versuch wird mit den in Beispiel 2 beschriebenen Stämmen durchgeführt. Die Herstellung der Mischkultur erfolgt so, dass eine Endverdünnung von $10^{-2}$, $10^{-3}$ und $10^{-4}$ in Tyrode erreicht wird.

Die Mischkulturen werden 5 Stunden bei 30°C im Schüttelwasserbad bebrütet und nach dieser Zeit Proben zur Bestimmung der Abtötung entnommen.

### Tabelle III

| Konzen-tration (mg/l) a.i. | Monobromdi-cyanomethan | | | Dibromdi-cyanomethan | | |
| | $10^{-2}$ | $10^{-3}$ | $10^{-4}$ | $10^{-2}$ | $10^{-3}$ | $10^{-4}$ |
|---|---|---|---|---|---|---|
| 2 | + | + | (+) | + | (+) | — |
| 5 | + | + | — | + | (+) | — |
| 10 | + | — | — | + | — | — |
| 30 | (+) | — | — | (+) | — | — |

+ = Wuchs, keine Abtötung
(+) = Wuchs, weniger als Kontrolle, > 10 Kolonien
— = kein Wuchs, alle Bakterien abgetötet

Aus den Tabellen I, II und III ist zu erkennen, dass die beiden Verbindungen eine hervorragende bakterizide Wirkung besitzen. 5 - 10 ppm der Verbindungen reichen aus, um die für die Kühlwasserbehandlung so wichtigen schleimbildenden Bakterien in 5 bzw. 24 Stunden abzutöten. In dem gleichen Konzentrationsbereich sind die Produkte auch in Weisswasser bei der Papierfabrikation gegen die dort vorhandenen Bakterien abtötend wirksam.

*Beispiel 4*

*Untersuchung der Wirksamkeit im Kühlturm*

Der im Labor untr Beleuchtung (14 h Licht- 10 h Dunkel-Wechsel) befindliche Kreislauf besitzt folgende technische Daten:

a) Volumen       27 l
b) Verdunstung:       320 ml/h
c) Abschlemmverlust:       0,8 l/h
d) Frischwasser=Verdunstung+Abschlemmverlust
e) Kühlleistung:       $\Delta T$ = 2°C von 26°C auf 24°C

In dem Kühlkreislauf, der mit Leitungswasser gespeist wurde, hatte sich eine natürliche Bakterienflora entwickelt.

Diese wurde mit 10 ppm Dibromdicyanomethan bzw. 5 oder 10 ppm Monobromdicyanomethan (5% Formulierung in Testbenzin) behandelt. Ausgewertet wurde der Versuch durch Bestimmung des Keimtiters mittels Verdünnen und Ausspalten jeweils direkt vor und nach verschiedenen Zeiten der Zugabe des Biocids.

### Tabelle IV

| Stunden nach Dosierung | Keimzahl/ml | | |
| | 10 ppm Di-bromdicya-nomethan | 10 ppm Mo-nobromcya-nomethan | 5 ppm Mo-nobromcya-nomethan |
|---|---|---|---|
| 0 | $4,5 \times 10^6$ | $2,3 \times 10^7$ | $5,6 \times 10^4$ |
| 1 | | $7,2 \times 10^1$ | $4,5 \times 10^1$ |
| 3 | $2,2 \times 10^2$ | $2,6 \times 10^3$ | $3,4 \times 10^2$ |
| 24 | | $4,3 \times 10^6$ | $6,4 \times 10^5$ |
| 28 | $5,9 \times 10^4$ | | |
| 48 | $1,0 \times 10^6$ | | |

Mit Dibromodicyanomethan ist in 3 h durch die Behandlung des Kühlturmes die Keimzahl um 4 Zehner-Potenzen gesunken, 28 h nach Behandlung sind erst ca. 1% der Keime wieder gewachsen.

Mit 5 ppm Monobromcyanomethan können noch 99,9% der Keime im Kühlkreislauf in 1 h getötet werden.

Monobromcyanomethan und Dibromdicyanomethan zeigt daher in dem Modellkühlturm eine hervorragende Wirkung, obwohl zu erwarten war, dass wegen der hohen Verdunstungsrate des Systems das Produkt sich schnell verflüchtigt.

*Beispiel 5*

*Untersuchung der Wirksamkeit in Kreisläufen*

Die im Freien stehenden (natürliche Sonneneinstrahlung, Staubeinfall, Witterungseinflüsse) Kreisläufe bestanden aus:

a) einem Kunststoff-Fass mit einem Volumen von 113 l und einem Überlauf
b) einer Pumpe (21 l/min bei 3 m Förderhöhe)
c) einem Kühlturm mit Oregon-(Splint), Oregon-(Kernholz), Eiche-Fichte-, Asbestzement- und PVC-Platte.

Die Frischwasser-Zufuhr war so eingestellt, dass der Spritz- und Verdunstungsverlust ausgeglichen wurde und die Biocide in 24 h ungefähr 1/2 verdünnt wurden.

Die Kreisläufe sind durch den natürlichen Staubeinfall und nicht durchgezielte Beimpfung infiziert.

Zur Verhinderung der Schleimbildung und des Algenwuchses wurde der Kreislauf mit 20 ppm Dibromdicyanomethan (25%ige Formulierung in Diäthylenglycoldimethyläther) 2mal pro Woche behandelt.

Ausgewertet wurde der Versuch durch Bestimmung des Keimtiters mittels Verdünnen und Ausspateln jeweils direkt vor Biocid-Zugabe und 3 h (die ersten 7 Tage, danach nur noch direkt vor Biocid-Zugabe) danach sowie durch visuelle Beobachtung des Bewuchses auf den Platten.

Tabelle V

| Zeit | Keimzahl/ml | Dosierung |
|---|---|---|
| 0 | $1,8 \times 10^4$ | + |
| 3 h | $1,8 \times 10^1$ | |
| 27 h | $9,8 \times 10^2$ | |
| 3 Tage | $3,3 \times 10^5$ | + |
| 3 Tage + 3 h | $9,8 \times 10^2$ | |
| 7 Tage | $2,7 \times 10^5$ | + |
| 7 Tage + 3 h | $2,6 \times 10^2$ | |
| 10 Tage | $1,6 \times 10^5$ | |

Nach jeder Behandlung mit 20 ppm Dibromdicyanomethan konnte die Keimzahl stark reduziert werden. Während des gesamten Versuchszeitraumes wurde keine Schleimbildung festgestellt, der Algenwuchs auf den Hölzern war im Vergleich zur Kontrolle ohne Behandlung stark reduziert.

*Beispiel 6*

*Wirkung gegen Desulfovibrio von Monobromcyanomethan + Dibromcyanomethan*

Die Wirkung gegen einen Nordsee-Stamm Desulfovibrio wurde in API Medium Nr. 38 getestet.

0,1 ml einer 14tägigen Kultur wurden in 10 ml frisches Medium gegeben und dem Biocid mit 5, 10, 20 und 50 ppm ausgesetzt. Nach 1 h bei 37°C wurde zur Bestimmung der Abtötung 0,1 ml entnommen und in 10 ml frisches Medium gegeben. Die Wuchshemmung sowie Abtötung wurde nach 14 tagen Bebrütung visuell bestimmt.

Tabelle VI

| Konzen-tration mg/l | Hemmung | | Abtötung | |
|---|---|---|---|---|
| | $BrCH(CN)_2$ | $Br_2C(CN)_2$ | $BrCH(CN)_2$ | $Br_2C(CN)_2$ |
| 5 | — | — | + | + |
| 10 | — | — | + | + |
| 20 | — | — | + | + |
| 50 | — | — | — | — |
| 0 | + | + | + | + |

+ = Wuchs       — = kein Wuchs

Monobromdicyanomethan bzw. Dibromdicyanomethan haben auch eine gute Wirkung gegen Desulfovibrio-Stämme.

*Beispiel 7*

*Wirkung von Dibromcyanomethan in Bohr- und Schneidölen*

Versuchsbeschreibung
Die Untersuchung fand mit folgendem Kühlschmierstoff statt:
Ein teilsynthetisches Bohröl mit einem Ölgehalt von weniger als 60% und u.a. Hochdruckzusätzen und nichtionogenen Emulgatoren. Die Versuchsapparatur wurde nach E.C. Hill, O. Gibbon, P. Davies, Biocides for use in oil emulsions, Tribology international June 1976, aufgebaut.

Das Konzentrat wurde mit $H_2O$ 1/20 verdünnt und dann der Schmierstoff mit einer Bakterien-Hefen-Mischkultur angeimpft (Endverdünnung 1/1000).

Die zum Animpfen dienende Mischkultur bestand aus folgenden Stämmen (1 : 1 : 1 usw. Verhältnis der ÜNK's):

Escherichia coli
Staphylococcus aureus
Pseudomonas aeruginosa
Enterobacter aerogenes
Proteus vulgaris
Pseudomonas oleovorans
Candida albicans
Endomyces geotrichum

Zur Auswertung des Versuches wurden vor dem wöchentlichen Animpfen und vor der Dosierung mit 50 mg/l Dibromdicyanomethan im Testbenzin (Dosierung 2-3 mal pro Woche) jeweils Proben entnommen und mittels Verdünnen und Ausspateln auf Caso-Pepton-Agar die Keimzahl bestimmt.

Aus folgender Tabelle VII ist deutlich die hervorragende Wirkung von Dibromdicyanomethan in diesem teilsynthetischen Bohröl zu erkennen. Trotz wöchentlichem Animpfen von Bakterien und Hefen, bleibt die Keimzahl unter $10^2$ Keime/ml Kontrolle: $> 10^7$ (K/ml).

Tabelle VII

| Zeit (Tage) | Kontrolle (K/ml) | Dibromdicyano-methan (K/ml) |
|---|---|---|
| 3 | $10^7$ | 10 |
| 5 | | 10 |
| 7 | $> 10^7$ | 30 |
| 10 | | 30 |
| 12 | | 30 |
| 14 | $> 10^7$ | 30 |
| 17 | | 10 |
| 19 | | $< 10$ |
| 22 | $> 10^7$ | $< 10$ |
| 26 | | 10 |
| 28 | $> 10^7$ | $< 10$ |
| 31 | | 20 |
| 33 | | 20 |
| 35 | $> 10^7$ | 30 |
| 38 | | 30 |
| 40 | | 40 |
| 42 | $> 10^7$ | 40 |
| 45 | | 60 |
| 47 | | 60 |
| 49 | $> 10^7$ | 80 |

**Patentansprüche**

1. Verwendung von Mono- oder Dibromdicyanomethan zur Bekämpfung von Schadorganismen in Wasser, in Wasser enthaltenden Systemen, in Ölen oder in Treibstoffen.

2. Verwendung nach Anspruch 1, dadurch gekennzeichnet, dass 1-100 ppm Mono- oder Dibromdicyanomethan eingesetzt werden.

3. Verwendung nach Anspruch 1, dadurch gekennzeichnet, dass 2-30 ppm Mono- oder Dibromdicyanomethan eingesetzt werden.

4. Verwendung nach Anspruch 1 in Kühlkreisläufen, Schneid- und Bohrölen, Papier- und Zellstoffmassen, wässrigen Dispersionsfarben, Pigmentdispersionen, Agrarchemikalien, Leimen und Stärkekleistern, sowie bei der sekundären Ölgewinnung.

5. Verwendung nach Anspruch 1 in Ölen und Treibstoffen.

6. Verwendung nach Anspruch 1 in Kühlkreisläufen.

7. Verwendung nach Anspruch 1 von Dibromdicyanomethan.

8. Verwendung nach Anspruch 1 von Monobromdicyanomethan.

9. Mittel zur Bekämpfung von Mikroorganismen, enthaltend Mono- oder Dibromdicyanomethan und einen Hilfsstoff, wobei die Konzentration des Dibrommalonitrils, bezogen auf das gesamte Mittel, 0,1 - 80 Gew.-% beträgt.

10. Mittel nach Anspruch 9, dadurch gekennzeichnet, dass es übliche zur Bekämpfung von Mikroorganismen geeignete Zusatzstoffe enthält.

11. Mittel nach Anspruch 9, enthaltend Mono- oder Dibromdicyanomethan und einen üblichen Trägerstoff.

12. Mittel nach Anspruch 11, enthaltend Mono- oder Dibromdicyanomethan und ein Lösungsmittel.

13. Mittel nach Anspruch 10, enthaltend als Zusatzstoff ein Detergens.

**Claims**

1. A method of controlling harmful organisms in water, in aqueous systems, in oils or in fuels, which comprises the use of mono- or dibromodicyanomethane.

2. A method according to claim 1, wherein mono- or dibromodicyanomethane is used in a concentration of 1 to 100 ppm.

3. A method according to claim 1, wherein mono- or dibromodicyanomethane is used in a concentration of 2 to 30 ppm.

4. A method according to claim 1, wherein harmful organisms are controlled in cooling circulation systems, cutting and drilling oils, paper and cellulose pulps, aqueous disperse dyes, pigment dispersions, agrochemicals, sizes and starch pastes, as well as in secondary oil recovery.

5. A method according to claim 1, wherein harmful microorganisms are controlled in oils and fuels.

6. A method according to claim 1, wherein harmful microorganisms are controlled in cooling circulation systems.

7. A method according to claim 1, which comprises the use of dibromodicyanomethane.

8. A method according to claim 1, which comprises the use of monobromodicyanomethane.

9. A composition for controlling microorganisms, which contains mono- or dibromodicyanomethane and an auxiliary, wherein the concentration of the dibromomalonitrile, based on the entire composition, is 0.1 to 80% by weight.

10. A composition according to claim 9, which contains conventional additives suitable for controlling microorganisms.

11. A composition according to claim 9, which contains mono- or dibromodicyanomethane and a conventional carrier.

12. A composition according to claim 11, which contains mono- or dibromodicyanomethane and a solvent.

13. A composition according to claim 10, which contains a detergent as additive.

**Revendications**

1. Utilisation du mono- ou du dibromodicyanométhane pour lutter contre des organismes nuisibles dans l'eau, dans des systèmes contenant de l'eau, dans des huiles ou dans des carburants.

2. Utilisation selon la revendication 1, caractérisée en ce qu'on utilise 1 à 100 ppm de mono- ou de dibromodicyanométhane.

3. Utilisation selon la revendication 1, caractérisée en ce qu'on utilise 2 à 30 ppm de mono- ou de dibromodicyanométhane.

4. Utilisation selon la revendication 1 dans des circuits de refroidissement, des huiles de coupe et de forage, des compositions de papier et de cellulose, des peintures aqueuses au latex, des dispersions de pigments, des produits chimiques pour l'agriculture, des colles et colles à l'amidon, ainsi que pour la récupération secondaire de l'huile de pétrole.

5. Utilisation selon la revendication 1 dans des huiles et carburants.

6. Utilisation selon la revendication 1 dans des circuits de refroidissement.

7. Utilisation selon la revendication 1 du dibromodicyanométhane.

8. Utilisation selon la revendication 1 du monobromodicyanométhane.

9. Produit pour lutter contre les micro-organismes, contenant du mono- ou du dibromodicyanométhane et un adjuvant, la concentration du dibromomalonitrile étant, par rapport au produit total, de 0,1 à 80% en poids.

10. Produit selon la revendication 9, caractérisée en ce qu'il contient des additifs convenables usuels pour la lutte contre les micro-organismes.

11. Produit selon la revendication 9, contenant du mono- ou du dibromodicyanométhane et un support usuel.

12. Produit selon la revendication 11, contenant du mono- ou du dibromodicyanométhane et un solvent.

13. Produit selon la revendication 10, contenant un détergent comme additif.